# EUROPÄISCHE PATENTANMELDUNG

(11) **EP 3 641 143 A1**
(43) Veröffentlichungstag der Anmeldung: **22.04.2020**
(21) Anmeldenummer: 18200967.0
(22) Anmeldetag: 17.10.2018
(51) Int. Cl.: H04B 5/00, H04B 5/02

(54) **SPULENANORDNUNG FÜR EIN PROGRAMMIERGERÄT UND PROGRAMMIERGERÄT**

(71) Anmelder: BIOTRONIK SE & Co. KG, 12359 Berlin (DE)
(72) Erfinder: MERLIN, Julian, 10713 Berlin (DE)
(74) Vertreter: Engelhardt, Björn

(57) **Zusammenfassung**

Die Offenbarung betrifft eine Spulenanordnung für ein Programmiergerät mit einer Sendespule (2), die eingerichtet ist, ein Sendesignal abzugeben, und mehreren Empfangsspulen (3a, 3b), die eingerichtet sind, ein Empfangssignal zu empfangen. Die Sendespule (2) und die Empfangsspulen sind derart angeordnet, dass die durch ein von der Sendespule (2) abgegebenes Sendesignal in den Empfangsspulen (3a, 3b) erzeugten magnetischen Flüsse sich im Wesentlichen gegenseitig aufheben oder die durch ein von der Sendespule (2) abgegebenes Sendesignal in den Empfangsspulen (3a, 3b) induzierten Spannungen sich im Wesentlichen gegenseitig aufheben. Des Weiteren ist ein Programmiergerät offenbart.

## Beschreibung

Die Offenbarung betrifft eine Spulenanordnung für ein Programmiergerät und ein Programmiergerät.

### Hintergrund

Einige medizinische Implantate (z.B. Herzschrittmacher oder Kardioverter-Defibrillatoren) können mit einem Programmiergerät kommunizieren, um beispielsweise Messwerte von dem Implantat an das Programmiergerät zu übermitteln und/oder Geräteparameter von dem Programmiergerät an das Implantat zu übertragen. Die Kommunikation erfolgt in der Regel mittels elektromagnetischer Felder.

Bei Kommunikationssystemen, die in beiden Richtungen arbeiten, müssen Sender und Empfänger entweder auf die gleiche oder auf verschiedene eng zusammenliegende Antennen arbeiten. Dies erfordert in der Regel Umschaltmechanismen oder andere Schutzvorkehrungen, um zu verhindern, dass die Sendeleistung in den Empfängereingang gelangt und dort verbraucht wird, anstatt abgestrahlt zu werden. Bei dicht zusammenliegenden magnetischen Loopantennen werden ohne Kompensation beim Senden in den Empfänger starke Felder eingekoppelt. Um den Empfänger zu schützen, müssen Schutzdioden verwendet oder andere Maßnahmen zur Spannungs- oder Strombegrenzung ergriffen werden, falls keine aktive Umschaltung erfolgen soll. Ferner ist es oft nötig, die Empfangsspulen elektrisch zu belasten, d. h. ausreichend niederohmig abzuschließen, um Spannungsüberhöhungen bei der Eigenresonanzfrequenz zu begrenzen. Diese Maßnahmen führen in der Regel zu einem nicht unerheblichen Abzug von Energie aus dem Sendefeld, wodurch die Reichweite reduziert wird. Durch Resonanzen in den Empfangsspulen können auch Rückwirkungen auf das Sendefeld erfolgen. In der Regel ist es ohne Kompensation der beim Senden induzierten Spannungen in den Empfangsspulen auch nicht möglich, gleichzeitig zu senden und zu empfangen, da der Empfänger hochgradig übersteuert wird.

Das Dokument US 5,630,835 A beschreibt einige Methoden um Sender und Empfänger zu entkoppeln. Dabei werden Spulen umgeschaltet oder abgeschaltet. In einer Ausführungsform weist eine Antenne zwei Spulen auf, die derart angeordnet sind, dass die Effekte von Fernfeld-Interferenzsignalen von empfangenen Nahfeldsignalen unterdrückt werden.

Das Dokument DE 199 28 216 C2 offenbart eine Telemetrie-Spulenanordnung zum Empfang von Datensignalen, insbesondere von kardiologischen Implantaten. Die Anordnung umfasst einen Mehrfach-Spulenkörper mit vier koaxial hintereinander angeordneten Spulen. Eine der vier Spulen ist eine Sendespule. Die anderen Spulen sind zu einem Empfangsspulensystem variabel verschaltbar.

Das Dokument US 5,741,315 A offenbart eine Vorrichtung zum Empfangen von Telemetriesignalen von einem Implantat. Eine Spule empfängt sowohl nutzbare Signalkomponenten als auch parasitäre Signalkomponenten. Eine Kompensationsspule wird zur Unterdrückung der parasitären Signalkomponenten verwendet.

Das Dokument US 8,847,434 B2 offenbart eine Antennenanordnung mit einer Kompensationsspule, um ein Sendesignal in einem Empfänger zu reduzieren.

### Zusammenfassung

Aufgabe ist, verbesserte Technologien für die Kommunikation zwischen zwei Geräten, insbesondere zwischen einem Programmiergerät und einem Implantat, anzugeben. Eine Einwirkung eines Sendesignals auf eine Empfangsschaltung soll verringert werden.

Es sind eine Spulenanordnung für ein Programmiergerät nach Anspruch 1 sowie ein Programmiergerät nach Anspruch 9 offenbart. Weitere Ausführungsformen sind Gegenstand von abhängigen Ansprüchen.

Nach einem Aspekt ist eine Spulenanordnung für ein Programmiergerät bereitgestellt. Die Spulenanordnung weist eine Sendespule auf, die eingerichtet ist, ein Sendesignal abzugeben, und mehrere Empfangsspulen, die eingerichtet sind, ein Empfangssignal zu empfangen. Die Sendespule und die Empfangsspulen sind derart angeordnet, dass die durch ein von der Sendespule abgegebenes Sendesignal in den Empfangsspulen erzeugten magnetischen Flüsse sich im Wesentlichen gegenseitig aufheben oder die durch ein von der Sendespule abgegebenes Sendesignal in den Empfangsspulen induzierten Spannungen sich im Wesentlichen gegenseitig aufheben.

Nach einem weiteren Aspekt ist ein Programmiergerät für ein Implantat mit einer hier offenbarten Spulenanordnung bereitgestellt.

Es kann auch ein System mit einem Programmiergerat und einem medizinischen Implantat vorgesehen sein. Das medizinische Implantat kann ein implantierbarer Herzschrittmacher, ein implantierbarer Kardioverter-Defibrillator (ICD - implantable cardioverterdefibrillator) oder ein implantierbarer Sensor sein. Das Programmiergerät kann eingerichtet sein, mit dem Implantat zu kommunizieren. Beispielsweise können Messwerte von dem Implantat an das Programmiergerät übertragen werden und/oder Geräteparameter können von dem Programmiergerät an das Implantat übermittelt werden.

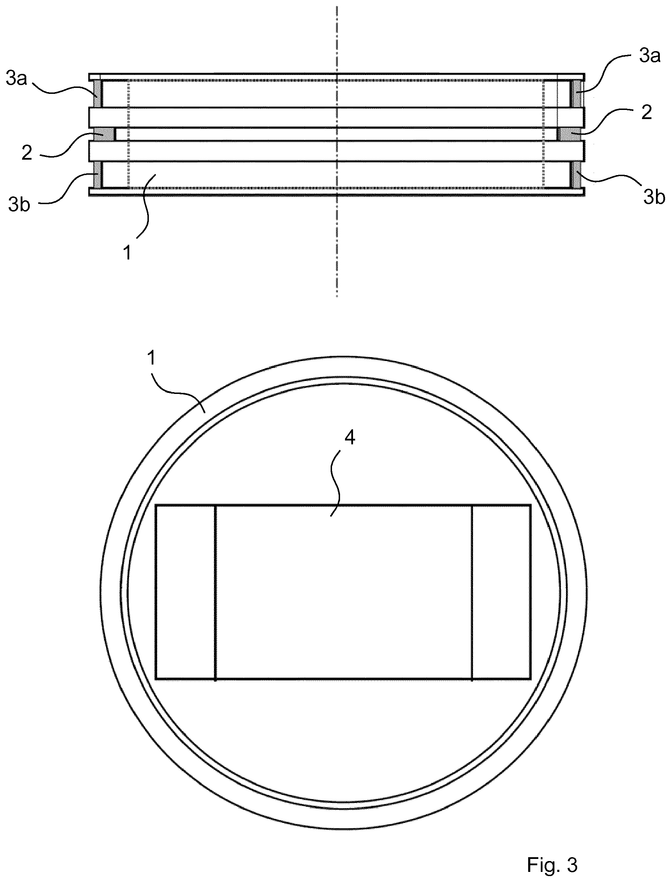

Die Empfangsspulen können ein Empfangsspulensystem bilden, das zwei, drei oder auch mehr Empfangsspulen umfasst. Die Empfangsspulen können miteinander gekoppelt (verschaltet) sein. Die Sendespule erzeugt beim Abgeben des Sendesignals ein elektromagnetisches Feld, das die Empfangsspulen des Empfangsspulensystems durchströmt. Die Sendespule ist derart angeordnet, dass der magnetische Fluss durch die gegenläufig verschalteten Empfangsspulen des Empfangssystems gleich groß ist und sich damit in Summe aufhebt bzw. bei unterschiedlichen Durchmessern und Windungszahlen der Empfangsspulen die sich induzierten Spannungen aufheben. Die Aufhebung ist durch mechanische Toleranzen beim Aufbau der Spulen und Wicklungen meistens nicht perfekt null. Für den magnetischen Fluss gilt: der Betrag der Summe des magnetischen Flusses durch die gegenläufig verschalteten Empfangsspulen ist wenigstens um einen Faktor 100 niedriger als der Betrag des magnetischen Flusses in der ersten Empfangsspule. Die am Empfänger, d.h. am Ausgang des Empfangsspulensystems, anliegende Spannung ist (z.B. bei der Ausführung nach Fig. 3) wenigstens um einen Faktor 100 niedriger als die in einer einzelnen Empfangsspule induzierte Spannung in gleichem Abstand zur Sendespule.

Mit der erfindungsgemäßen Spulenanordnung wird ein Verlust von Sendeleistung durch Einkopplung in Empfangsspulen vermieden. Des Weiteren wird eine starke Übersteuerung des Empfängers vermieden.

Das Empfangsspulensystem kann nach den Bedürfnissen des Empfangs ausgelegt werden, beispielsweise zur Unterdrückung von Fernfeldstörern und/oder zur Maximierung der Empfangsleistung in einer bestimmten Richtung (Richtcharakteristik).

Die Empfangsspulen können eine erste Empfangsspule und eine zweite Empfangsspule umfassen. Die erste Empfangsspule und eine zweite Empfangsspule können miteinander verschaltet sein. In einer Ausführungsform können genau zwei Empfangsspulen vorgesehen sein. Das Sendesignal der Sendespule induziert eine erste Spannung in der ersten Empfangsspule und eine zweite Spannung in der zweiten Empfangsspule. Die Sendespule, die erste Empfangsspule und die zweite Empfangsspule können derart angeordnet sein, dass eine Differenz zwischen der ersten Spannung und der zweiten Spannung nahezu null ist. Haben die erste Empfangsspule und die zweite Empfangsspule gleiche Form und gleiche Windungszahl, sind ein mit dem Sendesignal in der ersten Empfangsspule erzeugter erster magnetischer Fluss und ein mit dem Sendesignal in der zweiten Empfangsspule erzeugter zweiter magnetischer Fluss im Wesentlichen gleich groß und heben sich gegenseitig nahezu auf.

Die erste Empfangsspule kann auf einer ersten Seite der Sendespule angeordnet sein und die zweite Empfangsspule kann auf einer zweiten Seite der Sendespule angeordnet sein, wobei die erste Seite der zweiten Seite gegenüberliegt. Mit anderen Worten: Die Sendespule, die erste Empfangsspule und die zweite Empfangsspule können in drei voneinander verschiedenen Ebenen angeordnet sein. Die drei Ebenen können parallel zueinander sein. Beispielsweise kann die Sendespule mittig zwischen den beiden Empfangsspulen angeordnet sein, wobei die erste Empfangsspule oberhalb der Sendespule angeordnet ist und die zweite Empfangsspule unterhalb der Sendespule angeordnet ist.

Es kann vorgesehen sein, dass der Abstand zwischen der Sendespule und der ersten Empfangsspule und der Abstand zwischen der Sendespule und der zweiten Empfangsspule gleich groß sind. Alternativ können der Abstand zwischen der Sendespule und der ersten Empfangsspule sowie der Abstand zwischen der Sendespule und der zweiten Empfangsspule verschieden sein.

Gemäß einer anderen Ausführungsform kann vorgesehen sein, dass die Sendespule in einer ersten Ebene liegt und die erste Empfangsspule und die zweite Empfangsspule gemeinsam in einer zweiten Ebene liegen, wobei die erste Ebene von der zweiten Ebene verschieden ist. Die erste Ebene und die zweite Ebene können parallel zueinander sein.

Es kann ebenfalls vorgesehen sein, dass die Sendespule, die erste Empfangsspule und die zweite Empfangsspule gemeinsam in einer Ebene liegen.

Die erste Empfangsspule und die zweite Empfangsspule können koaxial angeordnet sind. Es kann vorgesehen sein, dass die Sendespule, die erste Empfangsspule und die zweite Empfangsspule koaxial angeordnet sind.

Liegen alle Spulen (Sendespule, erste Empfangsspule und zweite Empfangsspule) koaxial in einer Ebene, ist bei guter Fernfeldunterdrückung eine gute Sendesignalunterdrückung möglich, wenn die Sendespule wesentlich größer ist als die beiden Empfangsspulen. Das Feld im Inneren der Sendespule ist recht homogen, aber nicht perfekt homogen wie störende Fernfelder. Mit dieser Anordnung ist daher eine gleichzeitige optimale Unterdrückung der Sendefelder und Fernfelder schwer zu erreichen. Je größer die Sendespule im Vergleich zu den beiden Empfangsspulen ist, desto besser ist eine gleichzeitige Unterdrückung zu erreichen.

Die erste Empfangsspule und die zweite Empfangsspule können identisch sein, also insbesondere gleiche Windungszahlen, eine gleiche Form (z. B. kreisförmiger oder viereckiger Querschnitt), gleiche Dimensionen und gleiche Materialien aufweisen. Diese Ausführungsform kann als symmetrisches Doppelspulsystem bezeichnet werden. Die erste Empfangsspule und die zweite Empfangsspule können auch verschieden ausgebildet sein. In diesem Fall sind die Eigenschaften (Windungszahl, Form, Dimension und/oder Material) der beiden Empfangsspulen derart aufeinander abzustimmen, dass die beim Senden induzierten Spannungen in den Empfangsspulen sich gegenseitig (im Wesentlichen) aufheben. Die Windungszahlen der beiden Empfangsspulen werden derart ausgelegt, dass im Sendefall der Fluss in den beiden Empfangsspulen multipliziert mit den jeweiligen Windungszahlen gleich groß ist. Weitere Kriterien für die Empfangsspulen ergeben sich aus gewünschter Richtcharakteristik und Lage von Nullstellen. Weitere Ausführungen zu den Parametern der Empfangsspulen sind in dem Dokument DE 199 28 216 C2 beschrieben.

### Beschreibung von Ausführungsbeispielen

Im Folgenden werden beispielhafte Ausführungsformen unter Bezugnahme auf Figuren näher erläutert. Es zeigen:
- Fig. 1: eine perspektivische Ansicht eines Programmierkopfes,
- Fig. 2: eine Seitenansicht des Programmierkopfes aus Fig. 1,
- Fig. 3: eine Seitenansicht (obere Abbildung von Fig. 3) und eine Draufsicht (untere Abbildung von Fig. 3) einer Spulenanordnung,
- Fig. 4: für die Spulenanordnung nach Fig. 3 den Verlauf von Feldlinien beim Empfangen von Signalen von einem Implantat,
- Fig. 5: für die Spulenanordnung nach Fig. 3 den Verlauf von Feldlinien beim Senden von Signalen an ein Implantat,
- Fig. 6: eine Seitenansicht einer weiteren Ausführungsform einer Spulenanordnung,
- Fig. 7: für die Spulenanordnung nach Fig. 6 den Verlauf von Feldlinien beim Senden von Signalen an ein Implantat,
- Fig. 8: eine Seitenansicht noch einer anderen Ausführungsform einer Spulenanordnung,
- Fig. 9: eine Seitenansicht noch einer weiteren Ausführungsform einer Spulenanordnung,
- Fig. 10: eine Seitenansicht (obere Abbildung von Fig. 10) und eine Draufsicht (untere Abbildung von Fig. 10) einer anderen Ausführungsform einer Spulenanordnung und
- Fig. 11: eine Schaltskizze mit zwei Empfangsspulen und einer Sendespule.

Im Folgenden werden für gleiche Komponenten gleiche Bezugszeichen verwendet.

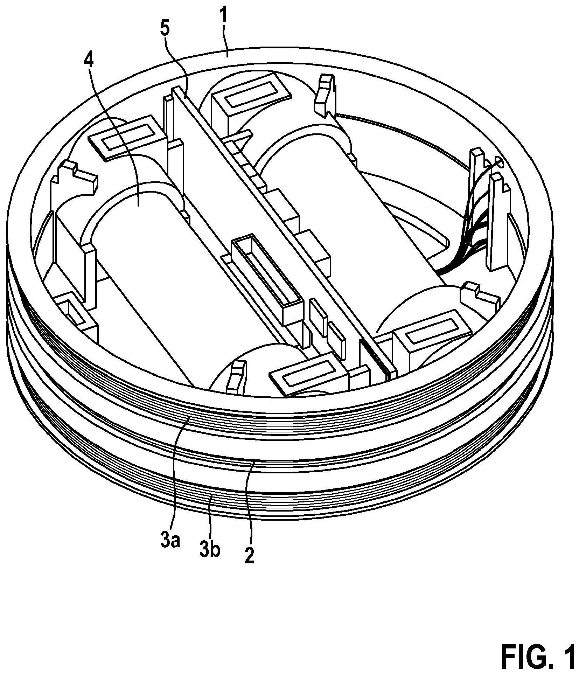

Fig. 1 zeigt einen Programmierkopf mit einer Spulenanordnung. An einem Spulenkörper 1 sind eine Sendespule 2, eine erste Empfangsspule 3a und eine zweite Empfangsspule 3b aufgewickelt. Die Spulen 2, 3a, 3b bilden jeweils einen Kreis. Im Inneren des Spulenkörpers 1 sind ein Magnet 4 (z. B. ein Permanentmagnet) und eine Leiterplatte 5 angeordnet. Auf der Leiterplatte 5 sind elektronische Komponenten (z. B. Prozessor und Speicher) für den Betrieb des Programmiergeräts angeordnet.

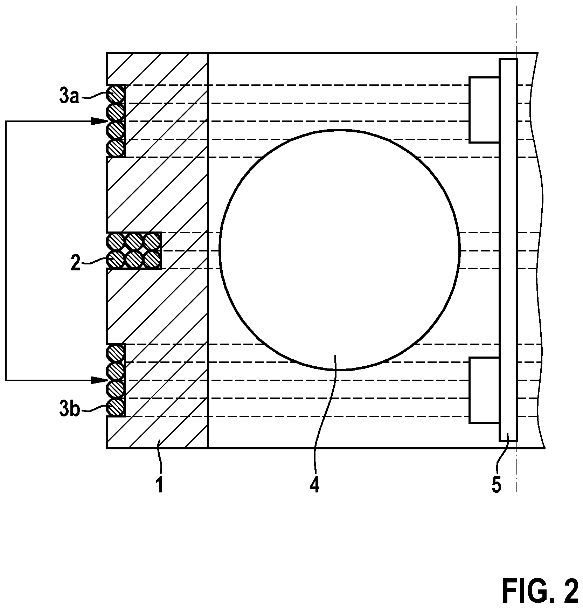

Die Sendespule 2 ist mittig zwischen der ersten Empfangsspule 3a und der zweiten Empfangsspule 3b angeordnet (Fig. 2). Der Abstand von der Sendespule 2 zu der ersten Empfangsspule 3a und der Abstand zwischen der Sendespule 2 und der zweiten Empfangsspule 3b sind gleich groß. Die Sendespule hat eine Windungszahl n = 7. Die erste Empfangsspule 3a und die zweite Empfangsspule 3b sind identisch aufgebaut. Beide Empfangsspulen 3a, 3b haben den gleichen Durchmesser und die gleiche Windungszahl (z. B. n = 40).

Aufgrund der Anordnung der Sendespule 2, der ersten Empfangsspule 3a und der zweiten Empfangsspule 3b werden beim Senden eines Signals von der Sendespule 2 in den beiden Empfangsspulen 3a, 3b zwei gleich große, entgegengesetzt gerichtete Felder erzeugt. Die Summe des Feldes in der ersten Empfangsspule 3a und des Feldes in der zweiten Empfangsspule 3b ist damit nahezu null. Abweichungen von dem exakten Wert null können sich aufgrund von Fertigungstoleranzen der Komponenten ergeben.

In Fig. 3 ist eine ähnliche Ausführungsform wie in Fig. 1 gezeigt. Die Sendespule 2 (z. B. mit einer Windungszahl n = 14) ist mittig zwischen zwei identischen Empfangsspulen 3a, 3b angeordnet. Die Empfangsspulen 3a, 3b können jeweils eine Windungszahl n = 60 haben, wobei zwei Lagen ä 30 Windungen aufgewickelt sind. Der Magnet 4 ist mittig in dem Spulenkörper 1 angeordnet.

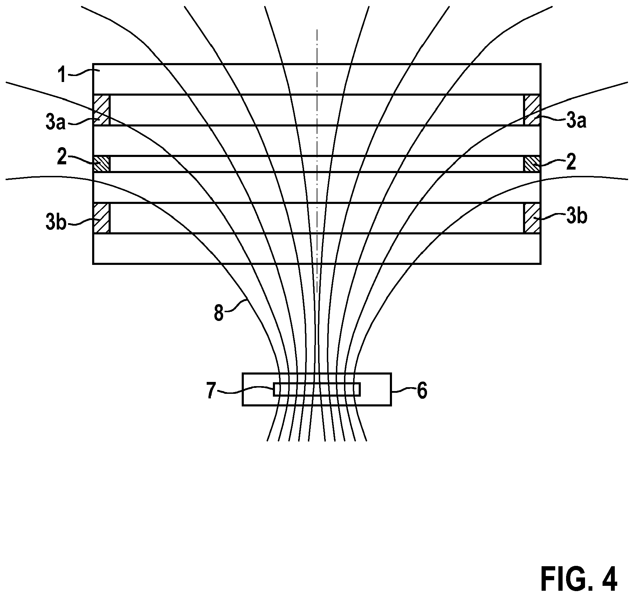

Fig. 4 zeigt schematisch den beispielhaften Verlauf der Feldlinien 8 für den Fall, dass ein Implantat 6 (mit einer Implantatspule 7) ein Signal an das Programmiergerät sendet. Dies ist der Empfangsfall für die Spulenanordnung. Die Flüsse in der ersten Empfangsspule 3a und in der zweiten Empfangsspule 3b sind unterschiedlich. Somit ist auch die in den beiden Empfangsspulen 3a, 3b induzierte Spannung unterschiedlich. Die Differenz der in den beiden Empfangsspulen 3a, 3b induzierten Spannung wird einem Empfänger (nicht dargestellt) zugeführt. Dies ist das sogenannte Doppelspulenprinzip.

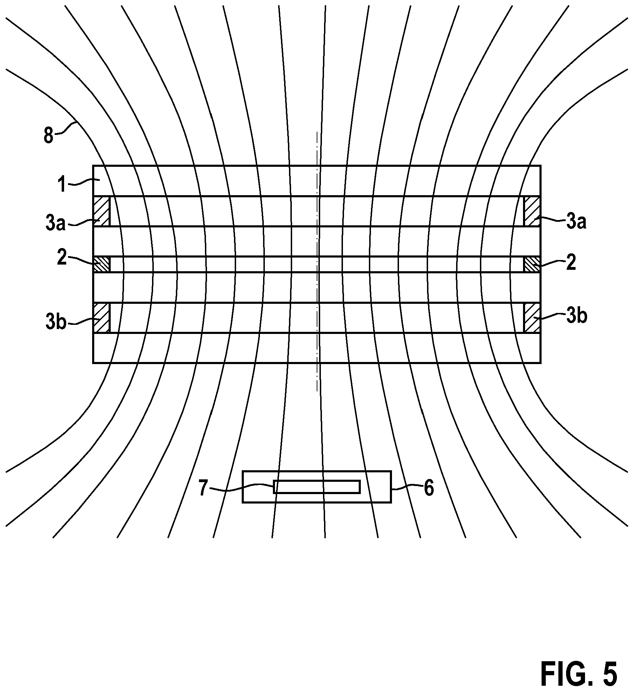

Fig. 5 zeigt schematisch die Feldlinien 8 für den Fall das mit der Sendespule 2 des Programmiergeräts ein Signal an das Implantat 6 gesendet wird (Sendefall). Aufgrund der mittigen Lage der Sendespule 2 sind der Fluss und die Spannung in den beiden Empfangsspulen 3a, 3b gleich groß und entgegengesetzt gerichtet. Die dem Empfänger zugeführte Differenzspannung ist null.

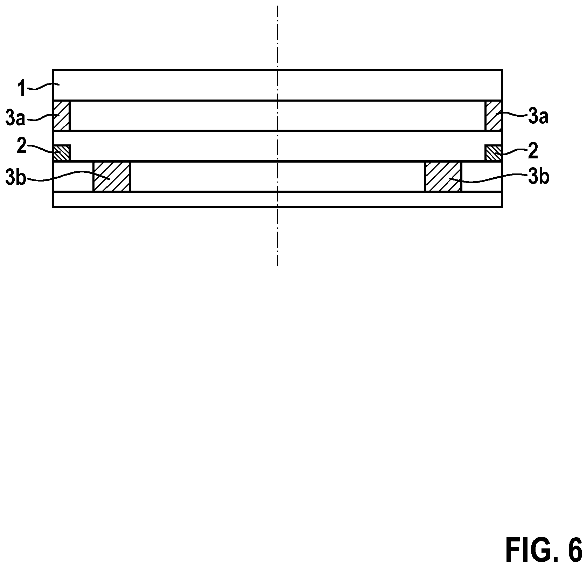

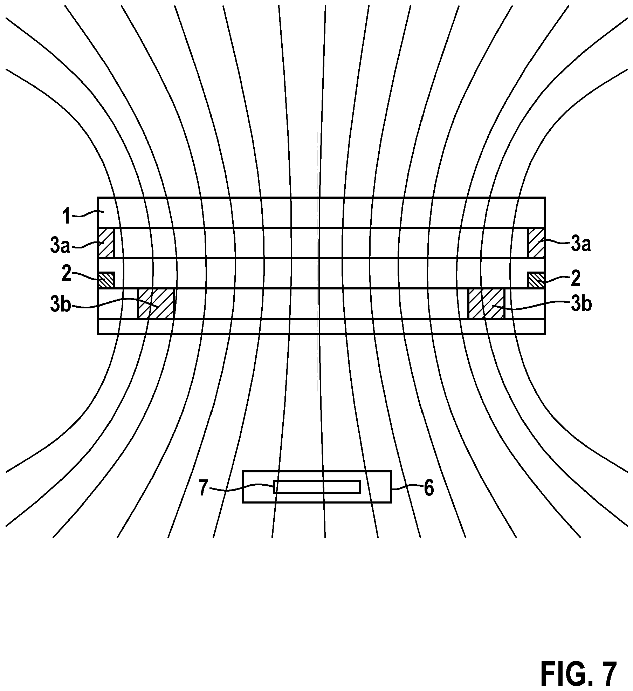

In Fig. 6 ist eine Ausführungsform mit asymmetrischen Empfangsspulen dargestellt. Die erste Empfangsspule 3a hat einen größeren Durchmesser als die zweite Empfangsspule 3b. Durchmesser und Windungszahlen der beiden Empfangsspulen 3a, 3b sind derart ausgelegt, dass im Sendefall der Fluss in den beiden Empfangsspulen 3a, 3b multipliziert mit den jeweiligen Windungszahlen gleich groß ist. Dadurch ist die dem Empfänger zugeführte Differenzspannung null. Beispielhafte Feldlinien 8 beim Senden an ein Implantat 6 sind in Fig. 7 gezeigt.

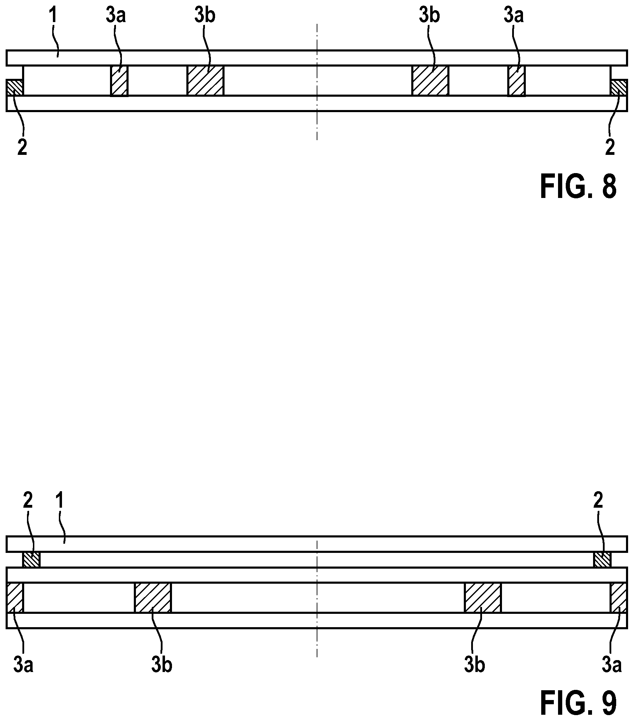

Fig. 8 zeigt eine Ausführungsform, bei der alle drei Spulen (Sendespule 2, erste Empfangsspule 3a und zweite Empfangsspule 3b) gemeinsam in einer Ebene angeordnet sind. Die erste Empfangsspule 3a hat einen größeren Durchmesser als die zweite Empfangsspule 3b. Der Durchmesser der Sendespule 2 ist größer als der Durchmesser der ersten Empfangsspule 3a. Die zweite Empfangsspule 3b hat eine höhere Windungszahl als die erste Empfangsspule 3a. Durchmesser und Windungszahlen der beiden Empfangsspulen 3a, 3b sind derart ausgelegt, dass im Sendefall der Fluss in den beiden Empfangsspulen 3a, 3b multipliziert mit den jeweiligen Windungszahlen gleich groß ist, oder dass der Fluss eines homogenen Fernfeldes in den beiden Empfangsspulen 3a, 3b multipliziert mit den jeweiligen Windungszahlen gleich groß ist. Bei dieser Anordnung kann durch Auslegung der Windungszahlen entweder eine optimale Fernfeldunterdrückung oder eine minimale Sendefeldeinkopplung oder ein Kompromiss erreicht werden.

Bei der in Fig. 9 dargestellten Ausführungsform sind die beiden Empfangsspulen 3a, 3b gemeinsam in einer ersten Ebene angeordnet und die Sendespule 2 ist in einer zweiten Ebene angeordnet, wobei die erste Ebene parallel zu der zweiten Ebene ist. Die erste Empfangsspule 3a hat einen größeren Durchmesser als die zweite Empfangsspule 3b.

Bei den in den Fig. 1 bis 9 dargestellten Ausführungsform sind die Sendespule 2, die erste Empfangsspule 3a und die zweite Empfangsspule 3b jeweils kreisförmig ausgeführt und koaxial angeordnet.

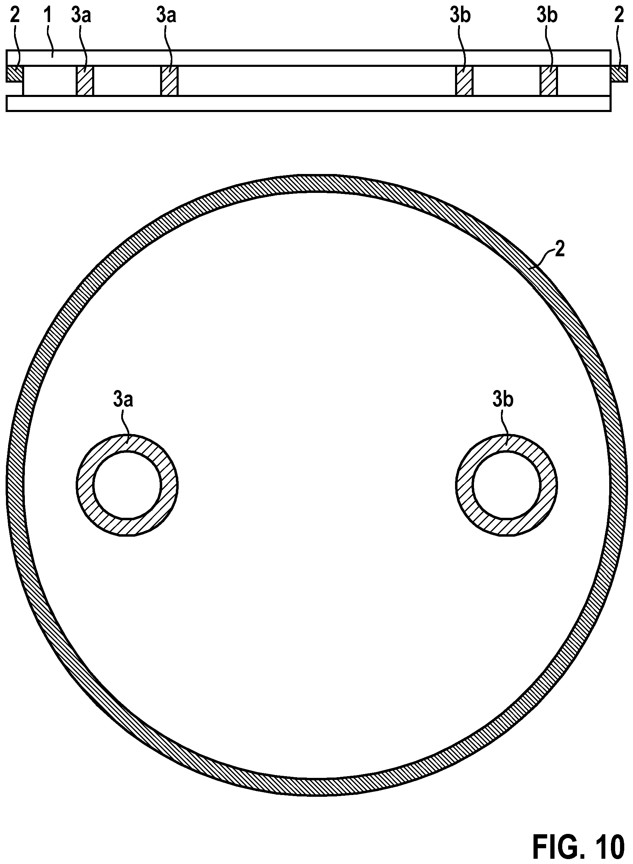

Eine weitere Ausführungsform ist in Fig. 10 gezeigt. Die Sendespule 2, die erste Empfangsspule 3a und die zweite Empfangsspule 3b sind gemeinsam in einer Ebene angeordnet. Die beiden Empfangsspulen 3a, 3b sind kreisförmig, jedoch nicht koaxial angeordnet. Beide Empfangsspulen 3a, 3b haben den gleichen Durchmesser und sind nebeneinander innerhalb der Sendespule 2 angeordnet.

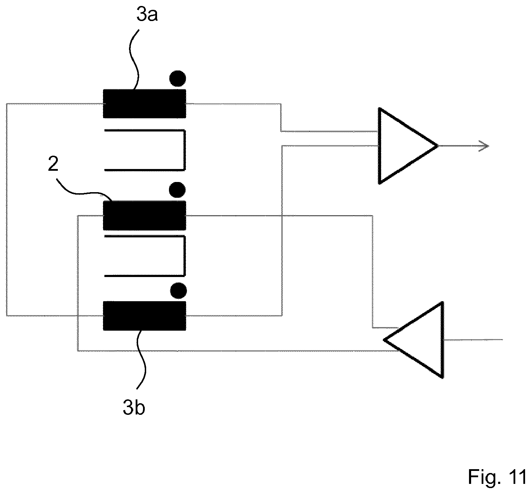

In allen offenbarten Ausführungsformen sind die erste Empfangsspule 3a und die zweite Empfangsspule 3b miteinander gekoppelt. Fig. 11 zeigt eine schematische Verschaltung der Sendespule 2, der ersten Empfangsspule 3a und der zweiten Empfangsspule 3b wie sie in allen offenbarten Ausführungsformen zum Einsatz kommen kann.

Mit den hier offenbarten Ausführungsformen können die folgenden Vorteile erzielt werden:
- Kompensation der Sendefelder am Empfänger ohne zusätzliche Kompensationsspulen oder Schaltungen,
- Ermöglichung bzw. Verbesserung des Voll-Duplex Betriebs (gleichzeitiges Senden und Empfangen) und/oder
- Empfangsspulen können elektrisch belastet werden, ohne das Sendefeld zu verzerren oder zu schwächen, und müssen im Sendefall nicht freigeschaltet werden.

Die in der Beschreibung, den Ansprüchen und den Figuren offenbarten Merkmale können für die Verwirklichung von Ausführungsformen sowohl einzeln als auch in beliebiger Kombination miteinander relevant sein.

### Bezugszeichenliste

- 1: Spulenkörper
- 2: Sendespule
- 3a: erste Empfangsspule
- 3b: zweite Empfangsspule
- 4: Magnet
- 5: Leiterplatte
- 6: Implantat
- 7: Implantatspule
- 8: Feldlinien

## Patentansprüche

1. Spulenanordnung für ein Programmiergerät mit
- einer Sendespule (2), die eingerichtet ist, ein Sendesignal abzugeben, und
- mehreren Empfangsspulen (3a, 3b), die eingerichtet sind, ein Empfangssignal zu empfangen,
wobei die Sendespule (2) und die Empfangsspulen derart angeordnet sind, dass
- die durch ein von der Sendespule (2) abgegebenes Sendesignal in den Empfangsspulen (3a, 3b) erzeugten magnetischen Flüsse sich im Wesentlichen gegenseitig aufheben oder
- die durch ein von der Sendespule (2) abgegebenes Sendesignal in den Empfangsspulen (3a, 3b) induzierten Spannungen sich im Wesentlichen gegenseitig aufheben.

2. Spulenanordnung nach Anspruch 1, wobei die Empfangsspulen eine erste Empfangsspule (3a) und eine zweite Empfangsspule (3b) umfassen.

3. Spulenanordnung nach Anspruch 2, wobei die erste Empfangsspule (3a) auf einer ersten Seite der Sendespule (2) angeordnet ist, wobei die zweite Empfangsspule (3b) auf einer zweiten Seite der Sendespule (2) angeordnet ist und wobei die erste Seite der zweiten Seite gegenüberliegt.

4. Spulenanordnung nach Anspruch 3, wobei der Abstand zwischen der Sendespule (2) und der ersten Empfangsspule (3a) und der Abstand zwischen der Sendespule (2) und der zweiten Empfangsspule (3b) gleich groß sind.

5. Spulenanordnung nach Anspruch 2, wobei die Sendespule (2) in einer ersten Ebene liegt, wobei die erste Empfangsspule (3a) und die zweite Empfangsspule (3b) gemeinsam in einer zweiten Ebene liegen und wobei die erste Ebene von der zweiten Ebene verschieden ist.

6. Spulenanordnung nach Anspruch 2, wobei die Sendespule (2), die erste Empfangsspule (3a) und die zweite Empfangsspule (3b) gemeinsam in einer Ebene liegen.

7. Spulenanordnung nach einem der Ansprüche 2 bis 4, wobei die erste Empfangsspule (3a) und die zweite Empfangsspule (3b) identisch ausgeführt sind.

8. Spulenanordnung nach einem der Ansprüche 2 bis 7, wobei die erste Empfangsspule (3a) und die zweite Empfangsspule (3b) koaxial angeordnet sind.

9. Programmiergerät für ein Implantat mit einer Spulenanordnung nach einem der vorangehenden Ansprüche.
